# EUROPEAN PATENT APPLICATION

(11) **EP 3 666 904 A1**
(43) Date of publication of application: **17.06.2020**
(21) Application number: 18212743.1
(22) Date of filing: 14.12.2018
(51) Int. Cl.: C12Q 1/6855

(54) **NUCLEIC ACID AMPLIFICATION AND IDENTIFICATION METHOD**

(71) Applicant: Lexogen GmbH, 1030 Vienna (AT)
(72) Inventor: GÖPEL, Yvonne, 1030 Wien (AT); MOLL, Pamela, 1200 Wien (AT); REDA, Torsten, 1140 Wien (AT); SEITZ, Alexander, 1140 Wien (AT)
(74) Representative: Sonn & Partner Patentanwälte

(57) **Abstract**

The present invention provides a method for generating labelled amplification fragments of a nucleic acid template comprising the steps of
providing said template nucleic acid,
annealing at least one oligonucleotide primer to said template nucleic acid,
elongating the at least one oligonucleotide primer in a template specific manner thereby creating an elongation product, wherein said elongating reaction stops when the elongation product reaches the 5' end of the template nucleic acid or a nucleic acid elongation stopper that is annealed to the template nucleic acid downstream of the elongation product,
providing an adaptor nucleic acid that comprises an identification sequence on its 5' end, wherein said identification sequence does not hybridize to the elongation stopper when in contact thereto,
ligating the adaptor nucleic acid at its 5' end to the 3' end of the elongation product, thereby generating a labelled amplification fragment.

## Description

The present invention relates to the field of nucleic acid analysis and amplification.

### Background

US 2010/0273219 A1 describes a method for multi-primer amplification for barcoding target nucleic acids.

WO 2012/134884 A1 describes barcoding template nucleic acids in a multiplex amplification reaction.

WO 2013/038010 A2 describes a method for generating an amplified nucleic acid part of a template nucleic acid using oligonucleotide primers and stoppers to prevent strand displacement and read-through by a polymerase that is used for generating the nucleic acid parts for sequencing. This method will remove biases during nucleic acid amplification.

WO 2014/071361 A1 describes a method for making dual barcoded nucleic acids using barcoded adaptor nucleic acids.

US 2014/0274729 A1 describes a method for generating cDNA libraries using DNA polymerases with strand displacement activity.

WO 2016/138500 A1 describes a method for barcoding nucleic acids for sequencing. Stochastic, i.e. random, barcodes are used as molecular labels.

Molecular labels, or unique molecular identifiers (UMIs), also called molecular barcodes, have been developed to identify PCR duplicates for reducing sequence specific PCR biases and for detecting rare mutations. Attaching unique molecular identifiers to RNA molecules, before any PCR amplification of a sequencing library preparation establishes a distinct identity for each input molecule. This makes it possible to eliminate the effects of a subsequent PCR amplification bias, which is particularly important where many PCR cycles are required, for example, when generating sequencing libraries from low template input amounts as in single cell studies. After PCR, molecules sharing the same sequence and also the same UMI are assumed to be identical copies derived from the same input molecule (Sena et al., Scientific Reports (2018) 8:13121).

### Summary of the invention

A goal of the invention is to provide an improved method of generating sequence fragments of a template nucleic acid which eases the allocation and assembly of said sequence fragments to a joined sequence that corresponds to the sequence of the template nucleic acid. A desired improvement would also reduce sequence bias during fragment generation and increase coverage of sequence fragments over the whole length of the template to increase confidence in the generated joined sequence.

Accordingly, the invention provides a method for generating labelled amplification fragments of a nucleic acid template comprising the steps providing said template nucleic acid, annealing at least one oligonucleotide primer to said template nucleic acid, elongating the at least one oligonucleotide primer in a template specific manner thereby creating an elongation product, wherein said elongating reaction stops when the elongation product reaches the 5' end of the template nucleic acid or a nucleic acid elongation stopper that is annealed to the template nucleic acid downstream of the elongation product, providing an adaptor nucleic acid that comprises an identification sequence on its 5' end, wherein said identification sequence does not hybridize to the elongation stopper when in contact thereto and preferably also not to the template, ligating the adaptor nucleic acid at its 5' end to the 3' end of the elongation product, thereby generating a labelled amplification fragment.

The invention also provides a method of for generating labelled amplification fragments of a nucleic acid template comprising the steps providing said template nucleic acid, annealing at least one oligonucleotide primer to said template nucleic acid, elongating the at least one oligonucleotide primer in a template specific manner thereby creating an elongation product, providing an adaptor nucleic acid that comprises an identification sequence, wherein said identification sequence does not hybridize to the template, ligating the adaptor nucleic acid preferably at its 5' end to the 3' end of the elongation product, thereby generating a labelled amplification fragment.

The invention further provides a kit suitable for performing the method. A kit of the invention may comprise at least one oligonucleotide primer capable of hybridizing to a template nucleic acid and priming an elongation reaction on its 3' end, one or more elongation stoppers capable of hybridizing to a template nucleic acid, preferably capable of priming an elongation reaction on its 3' end, one or more adaptor nucleic acids that comprise an identification sequence on its 5' end, wherein said identification sequence does not hybridize to the elongation stopper, preferably wherein the adaptor nucleic acid is bound to, hybridized to or is not bound to an elongation stopper, a reverse transcriptase, and an oligonucleotide ligase. The different components of the kit may be provided in different containers, such as vials.

The following detailed disclosure reads on all aspects, including methods and kits, and embodiments of the present invention. I.e. descriptions of methods may be a suitability of the kit. Any components described in the methods may be part of the kits. Components of the kit may be used in the inventive methods.

### Detailed description of the invention

The present invention provides a method for generating labelled amplification fragments of a nucleic acid template wherein an identification sequence is introduced as label before amplifying these fragments. A template nucleic acid can be present in multiple copies. According to the invention, fragmentation is usually a process that occurs during amplification, i.e. from a template of a given length, one or more (usually more) fragments are generated during amplification of parts of the template. The sequences of generated fragments may overlap when copies of templates generate at the same time fragments and the primers for synthesizing these complementary nucleic acid fragments anneal at different locations on different template copies. Although the inventive concepts work for a single fragment per template, preferably many fragments are generated from one template molecule, usually by using multiple primers that bind at different locations to the template.

The invention improves prior methods by binding an identification sequence to a generated fragment. Identification sequences can be introduced with the primer or after elongation, the synthesis of the complementary nucleic acid fragment. Then, the identification sequence is introduced by ligation of the elongation product with an adaptor nucleic acid. Surprisingly, the ligation reaction occurs with single stranded identification sequences, i.e. the parts of the identification sequences that have a non-hybridized (or "free") 5' end can ligate to the elongated product's 3' end. The ligation reaction usually involves a phosphate residue that is preferably provided on the 5' end of the identification sequence. Surprisingly, no template or stopper sequence dependent, supported by hybridization, vicinity of the adaptor nucleic acid to the 3' end of the elongation product is needed (as shown in the examples). Although such a vicinity can be supported by providing the adaptor nucleic acid with a complementary sequence part (downstream, i.e. 3' direction, of the identification sequence) for hybridization with an oligonucleotide that is bound to the template (also referred to herein as elongation stoppers or just stoppers, which may also be further primers in case more than one fragment per template is generated), a directed vicinity is not needed and can be the result of an undirected simple diffusion process. In particular, it has been shown that the adaptor nucleic acid can be ligated to an elongation product that has reached the 5' end of the template nucleic acid, and where no further downstream elongation stopper is present. Such a ligation reaction can occur to this end of the elongation product directly or after a polymerase has added one or more untemplated nucleotides based on its terminal transferase activity that some polymerases possess. This ligation to the elongation product that corresponds to the 5' end of the template has some surprising and beneficial advantages: It increases the occurrence of fragments at the 5' end of the template and therefore the sequence coverage increases fundamentally, which prior art methods lacked. In previous methods, the fragment start site distribution is constant which leads to a high coverage distribution by fragments in the middle of templates with much lower coverage, approaching zero, at its 3' and 5' ends (which is a result of number template copies, the average fragment size, and the sequencing read length). This effect on the 5' end is mitigated by the inventive method. Furthermore, the invention also provides embodiments to increase coverage on the 3' end of the template too.

The amplification fragments (generated as one fragment molecule per elongation reaction) are usually further amplified, i.e. copied. This means that the ligated identification sequence is amplified, hence copied, as well. Usually, the identification sequences are so manifold that a random selection process is capable of uniquely identifying a single fragments which carry the same sequence but result from different copies of one template. In all embodiments of the invention, the identification sequence helps to determine if fragment copies after sequencing are coming from different copies of the template because they have different identification sequences or if they are coming from the same template molecule and are just copies made during said further amplification.

A further method provides generating labelled amplification fragments of a nucleic acid template comprising the steps providing said template nucleic acid, annealing at least one oligonucleotide primer to said template nucleic acid, elongating the at least one oligonucleotide primer in a template specific manner thereby creating an elongation product, providing an adaptor nucleic acid that comprises an identification sequence, wherein said identification sequence does not hybridize to the template, ligating the adaptor nucleic acid preferably at its 5' end to the 3' end of the elongation product, thereby generating a labelled amplification fragment. This method is essentially the same as above and all preferred embodiments described herein apply as well, safe that a stopper is not used. Multiple primers, possibly without stopper function can be used. Adaptor nucleic acids can still be ligated to the elongation products after a diffusion process. For ligation, the elongation products can still be hybridized to the template or as single strand. However, preferably stoppers are used.

The inventive method starts with the step of providing said template nucleic acids. The template molecule is made accessible to a skilled practitioner for use in the inventive method. Usually the template is provided in a sample of nucleic acid molecules. Such template nucleic acids may be isolated from a cell, such as eukaryotic or prokaryotic cells. In particular preferred embodiments, the template is RNA. Total RNA or a fraction of RNA, such as mRNA or rRNA-depleted RNA of a cell can be provided. RNA amounts that are easy to handle are e.g. 0.1 pg to 500 ng, 1 pg to 200 ng, 10 pg to 100 ng, or 0.1 ng - 100 ng rRNA-depleted RNA or 0.1 ng to 1000 ng total RNA.In some embodiments, the amount of total RNA can e.g. 10 pg, and the amount of non-rRNA RNA can be below 1 pg. Primers, stoppers and adaptors are preferably DNA.

The method further comprises annealing at least one oligonucleotide primer to said template nucleic acid. An oligonucleotide primer is an oligonucleotide molecule, preferably DNA that anneals to the template and is capable for priming an elongation reaction as it is a standard practice in the art. The oligonucleotide primer (or simply "primer") preferably anneals to the template in at least one part of its length of e.g. 4 nucleotides to 30 nucleotides (nt) in length. Annealing is by hybridization. The primer may have a part that does not anneal to the template. Such further parts may be used to anneal to other oligonucleotides and/or be used for the further amplification mentioned above when amplification fragments are further amplified to produce copies thereof. Such further parts or portions may thus have a sequence to which other primers bind for this amplification/copying reaction. Such a part is also referred to as primer linker sequence. A primer linker sequence preferably has 4 nt to 30 nt in length.

Returning to the main inventive method, the at least one oligonucleotide primer is elongated in a template specific manner thereby creating an elongation product (complementary sequence). Such reactions are standard in the art and usually make use of a polymerase. If the template is RNA, then an RNA-dependent polymerase is used, such as a reverse transcriptase. If the template is DNA, then a DNA-dependent polymerase is used. The elongating reaction stops when it reaches a nucleic acid elongation stopper that is annealed to the template nucleic acid downstream of the elongation product or when the elongation product reaches the 5' end of the template nucleic acid. Obviously, when the elongation reaction reaches the 5' end of the template and thus runs out of template, it stops. Some polymerase may add one or more non-templated nucleotides at this point to the elongation product, which is acceptable or may even be beneficial when selecting for 5' coverage product in the sequence analysis of the produced labelled amplified fragments. This addition of non-templated nucleotides is however not necessary. Elongation reactions also stop when the elongation reaction reaches a nucleic acid elongation stopper that is annealed to the template nucleic acid downstream of the elongation product. Such a stopped reaction is described in length in WO 2013/038010 A2 (incorporated herein by reference). In this WO-document, the elongation stopper is referred to as "oligonucleotide stopper" or "further oligonucleotide primer". According to the present invention one term is used, i.e. nucleic acid elongation stopper or just "elongation stopper" or just "stopper". This inventive stopper can also be a primer and then corresponds to the "further oligonucleotide primer" of WO 2013/038010 A2. In essence, such a stopper stops the elongation reaction of an upstream elongation reaction (hence, the stopper is downstream of the elongation reaction) by presenting an obstacle on the template. The stopper is annealed or hybridized to the template and the elongation reaction does not displace the stopper and thus aborts. Read-through, i.e. displacement of a stopper would be a side reaction. Measures to prevent displacement of the stopper are described in length in WO 2013/038010 A2 and these can be used according to the invention. Briefly, preferred methods and means to prevent displacement of the stopper (due to stand displacement activity) are using an elongation stopper that comprises one or more modified nucleotides that increase the melting temperature in an annealing sequence for annealing to the template (a part of the stopper that anneals/hybridizes to the template). Increase in melting temperature refers to an unmodified, natural nucleic acid, such as DNA or RNA. Such modifications are e.g. LNA (locked nucleic acid), ZNA (zip nucleic acids), 2' fluoro nucleosides / 2' fluoronucleotides or PNA (peptidic or peptide nucleic acid). Other measures are using a polymerase that does not have strand displacement activity or using intercalators. Preferably 1, 2, 3, 4, 5 or 6 nucleotides are modified. Preferably the modified nucleic acids are on the 5' side of the sequence part of the stopper that hybridizes to the template. There may be further parts of the stopper in 5' direction that do not hybridize - such as amplification sequences that act the same as described for the oligonucleotide primer described above for amplification/copying in a further amplification reaction ("primer linker sequence") - in fact, such a further part is preferred for binding/hybridizing to the adaptor nucleic acid - see below. The adaptor may bind/hybridize to the "primer linker sequence" or to another part of the oligonucleotide stopper. In preferred embodiments, the elongation stopper and preferably also the oligonucleotide primer comprise(s) one or more modified nucleotide(s) that increase the melting temperature in an annealing sequence (linker) for annealing to the template.

Preferably, after the elongation reaction, the primers and stoppers that are not bound to the template are removed in a purification step. I.e. the elongation products hybridized to the template are purified and retained for further processing. Other embodiments of the invention are done in a single volume without purification. Such a purification can be done by methods known in the art, e.g. immobilisation of the template or elongation products to a solid phase (e.g. beads) and washing to remove any unbound primers and stoppers. An example method is solid phase reverse immobilization (SPRI; DeAngelis et al., Nucleic Acids Research, 1995, 23(22): 4742-4743).

The inventive method comprises the step of providing an adaptor nucleic acid that comprises an identification sequence on its 5' end. The 5' end is the end that is intended for ligation to the 3' end of the elongation product for the latter's labelling by the identification sequence. The identification sequence shall not hybridize to the elongation stopper or to the template. Thus, it is usually single stranded and not hybridized. Herein the term "identification sequence" is used for the 5' terminal part of the adaptor nucleic acid that is not hybridizing or annealing - even if only parts of the identification sequence would later be used for identification. Other parts of the adaptor nucleic acid may form a hybrid with, or anneal to, the elongation stopper. The adaptor nucleic acid may also comprise a complementary primer sequence, which is the target for a further amplification reaction of the labelled amplification fragments as mentioned above (called adaptor linker sequence). The identification sequence can be prevented from hybridization to the elongation stopper or to the template by selecting a sequence for the identification sequence that has no complement on the elongation stopper. It is also possible to select the identification sequence so that it has no complement on the template. This can be easily done if the sequence of the template is known. If it is unknown but from a biological source, then the identification sequence can be selected from sequences that do not or rarely occur in biological nucleic acids. Such sequences are known from "spike-in" nucleic acids, such as ERCC (External RNA Control Consortium) sequences or SIRV (spike-In RNA variants) sequences (see e.g. ERCC, BMC Genomics 2005 6: 150; Jiang et al., Genome Res. 2011, 21(9): 1543-1551; WO 2016/005524 A1, all incorporated herein by reference). If the identification sequence would anneal to the template in a side reaction, then this situation would usually prevent ligation in the next step and thus not lead to a labelled fragment and is thus not seen as result. Such side reactions can be tolerated but are not preferred. The easiest and most preferred method to prevent annealing of the identification sequence (and preferably the entire adaptor nucleic acid) to the template is by simply providing the adaptor nucleic acid after the elongation reaction. After the elongation reaction, the template is in form of a double strand with the elongation products (and the primer and stoppers). In this form the adaptor nucleic acid cannot bind to the template anymore since the template is already covered by hybridization partners. In this preferred method, the identification sequence may even have a sequence that is a complement to the template and may be capable to hybridize to the template but is hindered to do so by the succession of method steps. So, no consideration to template sequences is needed in this embodiment.

The most preferred option to prevent annealing of the identification sequence to the stopper is that parts of the stopper and parts of the adaptor carry complementary sequences to each other. Because at an approach of adaptor to the stopper the complementary sequences hybridize first and the identification sequence remains single stranded.

The inventive method further comprises ligating the adaptor nucleic acid at its 5' end to the 3' end of the elongation product, thereby generating a labelled amplification fragment. Ligation is usually performed using a ligase enzyme. The type of ligase depends on the nature of the oligonucleotides to be ligated and can be selected by a skilled practitioner. Example ligases include a DNA ligase or an RNA ligase. The ligase may also be an RNA ligase, especially an RNA ligase that has DNA ligating activity such as T4 RNA ligase 2. Further ligases are T4 DNA ligase, T4 RNA ligase 1, DNA ligase I, DNA ligase III, DNA ligase IV, E. coli DNA ligase, ampligase DNA ligase, truncated Rnl2, Rnl2 truncated K227Q, Thermus scotoductus ligase, Methano-bacterium thermoautotrophicum RNA ligase, thermostable Appligase (NEB), Chlorella virus DNA ligase or SplintR ligase. The ligase may be a single strand ligase or a double strand ligase. Also possible are combinations of ligases for different reactions in one reaction volume to be performed in parallel, e.g. when different elongation products and/or adaptor nucleic acid molecules are present and shall be ligated at the same time. Preferred combinations are DNA ligase and RNA ligase or a single strand ligase and a double strand ligase. The ligase reaction usually involves a phosphate residue that is preferably provided on the 5' end of the identification sequence of the adaptor nucleic acid. Also other 5' moieties can be used for ligation, e.g. ligation of adenylated ends. Such can be ligated with truncated ligases or App-ligases.

The generated labelled amplification fragments will have the structure from 5' to 3' after the ligation of: primer sequence - elongation product sequence - adaptor sequence with identification sequence bordering the elongation product sequence. The primer sequence may have a "primer linker sequence" and/or the adaptor sequence may have an "adaptor linker sequence". The products of the inventive method, i.e. the generated labelled amplification fragments, are preferably further amplified. Such a further amplification produces copies of the generated labelled amplification fragments by methods known in the art, such as PCR (polymerase chain reaction) or linear amplification. Such a further amplification usually involves the use of further primers that bind to the labelled amplification fragments, preferably on linker sequences, especially linker sequences located on the fragments ends, i.e. within the parts of the primer sequence and the adaptor sequence, in particular preferred on the 5' end of the primer sequence and the 3' end of the adaptor sequence. As mentioned above with regard to these primers and adaptors, they may have regions of known sequence to bind such primers of the further amplification ("primer linker sequence" and "adaptor linker sequence"). These regions (or "parts") may be so long and specific to not bind to the template; they may be universal primer binding sites, i.e. not selective between different adaptors/primers - contrary to the identification sequence, which is preferably unique.

The identification sequence provides a unique label for an amplification fragment and is therefore also referred to as unique molecular identifier (UMI) herein. The identification sequences can identify replicates of the further amplification (e.g. PCR) and reduce the effects of sequence dependent amplification bias. In preferred embodiments, the identification sequences are oligonucleotides with, mostly, random nucleotide distribution at each position which are ligated to extension products (fragments) prior to further amplification. If identification sequences are evenly distributed and their number is considerably larger than the number of identical extension products, then it is unlikely that the same identification sequence is ligated to two identical extension products (different copies). In this case, the number of distinct identification sequences after further amplification is the same as the number before further amplification. Identification sequences of the invention can also be used as described for UMIs in Sena et al. (Scientific Reports (2018) 8:13121). The entire sequence or parts of the entire sequence of the labelled fragment may be considered as a "read" in next generation sequencing methods and further sequence analysis. One or more reads are assembled during data analysis to obtain a joined sequence of the template. Subsequently, data analysis may also become a quantitative analysis of template molecules and fragments, which may provide insights if particular template copies are over or underrepresented, which e.g. hints are different expression rates of RNA splice variants. In preferred embodiments, the present invention further comprises the step of assembling the sequences of amplification fragments which are unique, wherein the labels are used to identify unique amplification fragments. Different identification sequences in the amplified labelled amplification fragments identify unique amplification fragments. The identification sequences enable duplicate and replicate identification and removal in the assembly or any other data analysis step.

In preferred embodiments, the identification sequence is 3 nt (nucleotides) or more in length, preferably 3 nt to 20 nt, especially preferred 4 nt to 15 nt or 5 nt to 10 nt, such as 3 nt, 4 nt, 5 nt, 6 nt, 7 nt, 8 nt, 9 nt, 10 nt, 11 nt, 12 nt, 13 nt, 14 nt, 15 nt or more in length. Such lengths are sufficiently small for easy handling and efficient ligation reactions but still provide a sufficiently large amount of different identification sequence due to nucleotide permutations in their nucleotides to provide the desired identification of single amplified fragments, preferably to provide unique labels thereto.

In preferred embodiments, in case the elongation product reaches the 5' end of the template nucleic acid, a nucleotide polymerase is allowed to add non-templated nucleotides to the elongation product, preferably by a terminal transferase activity of the polymerase, and/or preferably wherein 1 to 15 untemplated nucleotides are added in at least 70% of the extension products. As said above, such non-templated nucleotide addition is a property of some polymerases (see Chen et al. Biotechniques 2001, 30(3):574-582). This activity is most prominent in reverse transcriptases, such as M-MLV (murine leukemia virus) reverse transcriptase or AMV (alfalfa mosaic virus) reverse transcriptase. These non-templated nucleotides are usually of any nucleotide type (A, T(U), G, C) and may appear random. This means that elongation products of 5' ends of different templates may share the same sequence corresponding to the 5' end but then may continue by different, seemingly random further nucleotides that are the product of such non-templated addition. These different additions may be used to identify the exact position of the 5' end of the template sequence at the transition between the templated repeating sequence and the non-templated random additions. After the non-templated nucleotides, the labelled fragment continues with the identification sequence, which may be used as described above. In case the identification sequence is (also) random, the non-templated random nucleotides may be treated like a part of the identification sequence. The position of the identification sequence relative to the constant part the adaptor sequence identifies unambiguously the identification sequence.

In particularly preferred embodiments, a plurality of adaptor nucleic acids is provided and used in the ligation step. These adaptors of the plurality may have different identification sequences. This allows unique identification of the adaptors and the generated fragments to which they are ligated. Preferably at least 10, more preferred at least 50, or even 100 or more or 200 or more, adaptor nucleic acids with different identification sequences are provided and used in the ligation step. In particular preferred, as many adaptors with different identification sequences are used as different generated fragments with the same sequence are expected - or preferably more adaptors with different identification sequences. The expectation of the number of template copies can be based on the sample type, e.g. whole cell RNA, whole cell mRNA (transcriptome), amount of the RNA, and the complexity of the sample (how many different transcript variants are targeted which can be either the whole transcriptome or just selected genes or transcripts as it is the case in gene panels), etc.

In particular preferred, the identification sequence is a random sequence. "Random sequence" is to be understood as a mixture of different sequences with a high variance due to a random synthesis of at least a part of the identification sequence. Random sequences potentially cover the entire combinatory area for said sequence for 4 naturally occurring nucleotides (A, T(U), G, C). The random sequence may cover 1, 2, 3, 4, 5, 6, 7, 8, 9, 10, 11, 12, 13, 14, 15 or more nucleotides which are randomly selected from A, G, C or T (U). In terms of hybridizing capability of sequences of nucleotides T and U are used interchangeably herein. The full combinatory possible area for a random sequence portion is mⁿ, wherein m is the number of nucleotide types used (preferably all four of A, G, C, T(U)) and n is the number of the random nucleotides. Therefore, a random hexamer, wherein each possible sequence is represented, consists of 4⁶ = 4096 different sequences. The identification sequence shall not bind to the template. In all cases, but especially for random identification sequences, it is preferred to add the adaptor nucleic acid(s) after the elongation reaction. When the elongation product has reached the stopper (or end of the template) and essentially the entire template is then in form of a double strand with the elongation products, then the adaptor nucleic acid(s) are prevented from binding to the template.

In further embodiments of the invention, the primers and stoppers are selected to bind to on or more particular target sequences of interest in a template nucleic acid (with the stopper being downstream for an elongation product) so that an elongation sequence of a particular template part is obtained. Such targeting of specific regions is preferably used for transcripts (RNA) or genes (gDNA) as templates. Identification sequences are especially helpful when used in gene panels. Such as for the analysis of sequence variants of different species of templates, such as splice variants or other varying template sequences.

In especially preferred embodiments of the invention for all its embodiments and aspects, the elongation stopper has primer activity and is also elongated during the elongating step. This means that more than one primer is used and most have stopper function (i.e. prevent displacement - see above). Using several primers means that a template yields many generated fragments, i.e. coverage is improved. Although primers bind to one template each they will provide comprehensive coverage when different primers bind to different locations on the template. The inventive method using a plurality of primers (that preferably also are stoppers) will increase coverage since a new extension product will start at a position on the template where an upstream elongation product has just stopped. This yields many fragments that cover the entire template. Further, it means also that stoppers/primers (in this embodiment used synonymously) are used that bind to different parts of a template molecule. In general, binding to the template molecule is determined by the annealing sequences of the primers and stoppers. This sequence hybridizes with the template and may be varied to bind to different locations on the template. Preferably at least 9, at least 10, more preferred at least 49, at least 50, e.g. 100 or more or 200 or more, elongation stoppers are used that have different annealing sequences for annealing to the template. Thereby they will potentially anneal to different locations on the template nucleic acid. Preferably the annealing sequence is a random sequence. Random sequences are described above with regard to the identification sequence and the same applies to the annealing sequence of the primer, stopper and stoppers with primer function as well. Preferably, the random sequence of the annealing sequence may cover 1, 2, 3, 4, 5, 6, 7, 8, 9, 10, 11, 12, 13, 14, 15 or more nucleotides which are randomly selected from A, G, C or T (U).

Preferably the adaptor nucleic acid(s) is/are bound to, hybridized to, or is/are not bound to the elongation stopper(s). Such a binding reaction, e.g. by chemical reaction, complex formation or hybridization, facilitates positioning of the adaptor nucleic acid near the 3' end of the upstream elongation product to which its identification sequence that itself is not hybridzed to the stopper or template, and which surprisingly is not required for the ligation reaction to work. Preferably when the adaptor nucleic acids are bound to or hybridized to the elongation stoppers, then the identification sequence is selected independent of an annealing sequence of the elongation stopper for annealing the elongation stopper to the template. Both the annealing sequence and the identification sequence can be random sequences, preferably independently selected from each other. This is usually guarantied when the nucleic acid parts of the stopper and of the adaptor are universal sequences, i.e. any adaptor can bind to any stopper (which is preferred for all embodiments of the invention) and further an adaptor nucleic acid is not provided bound to the stopper, e.g. when the adaptor is provided only after the elongation reaction. In other embodiment or other parts of the reaction, they are not bound, such as when the elongation reaction reaches the 5' end of the template, where no stopper is usually hybridzed to because the stopper needs at least a minimal annealing sequence on the template, which moves the most downstream stopping position several nucleotides upstream from the 5' end. The adaptor is also able to be ligated to the elongation product without bind or hybridization to the elongation stopper. However, it is preferred in all embodiments that when the adaptor nucleic acid is ligated to the elongation product, said elongation stopper and/or the elongation product, in particular preferred its 3' end, is still hybridized to the template. It is also preferred that the adaptor nucleic acids are hybridized to the elongation stopper, especially preferred, after the elongation reaction and/or - in particular preferred - for ligation.

In preferments of the inventive method and kit, the oligonucleotide primer - and preferably but not necessarily also the elongation stopper - comprises a universal amplification sequence ("primer linker sequence", see above) and/or wherein the adaptor nucleic acid comprises a universal adaptor amplification sequence ("adaptor linker sequence", see above). Such an amplification sequence or "linker" can be used to bind primers for a further amplification as already mentioned above. A universal sequence means that it is the same for all primers, stoppers or adaptors, respectively. This allows binding of the same primer type to these oligonucleotides. In especially preferred embodiments, the universal amplification sequence (linker sequence) is also the same for the primers, stoppers and adaptors, i.e. a further amplification primer may bind to oligonucleotide primers, elongation stoppers and adaptor nucleic acids likewise. This facilitates easy handling since only one type of primer is necessary for further amplification. In other embodiments, the primers, stoppers and adaptors have different universal amplification sequences (linker sequences), i.e. a further amplification primer may only bind to oligonucleotide primers, another further amplification primer may only bind to elongation stoppers and a further amplification primer may only bind to adaptor nucleic acids. Within these groups, the primers are preferably universal. This still allows easy handling but better control since primers for both ends of labelled fragment will be different and can be selected specifically.

In preferred embodiments, a special oligonucleotide primer is used to select for and anneal to a selected sequence of the template, preferably on the 3' end of the template. In case of mRNA, or any other type of RNA that comprises an oligo(A) tail, such a 3' end can be annealed to with a complementary oligonucleotide primer, e.g. which comprises an oligo(dT) annealing sequence that is complementary to said oligo(A) tail. Preferably at least one oligonucleotide primer comprises an annealing sequence for annealing to a selected sequence of the template, which may be at or near the 3' end of the template. Such a selected sequence is any known sequence of the template, like an oligo(A) tail, but any other sequence when known can be used as well. Preferably, the oligonucleotide primer for the selected sequence comprises an oligo(dT) sequence for annealing to an oligo(A) sequence in the template. Preferably said oligo(dT) sequence comprises one or more 3' anchoring nucleotides different from the oligo(dT) sequence. This allows proper localization and binding to the 5' end of the oligo(A) template sequence. The anchoring nucleotide will anneal to the next non- A (e.g. T, G, C) on the template next to the oligo(A) part. If the next non-A nucleotide is unknown, it is possible to use a mixture of oligonucleotide primer with different anchoring primers, e.g. using three oligonucleotide primer with each non-T (e.g. A, G, C) nucleotide (complementary to the next non-A (e.g. T, G, C) on the template). In preferred embodiments, two anchoring nucleotides are used. The anchoring nucleotide next to said non-T nucleotide may selected from any nucleotide type (A, T(U), G, C) as it is not bordering the oligo(T). Said special oligonucleotide primer may not be stopper and may not comprise a sequence for hybridization to an adaptor since these are not needed if the special oligonucleotide primer anneals to or near the 3' end of the template - this means that no upstream elongation product will arrive at its position. Of course, for ease or unity in primer/stopper manufacturing such a sequence and/or stopper function may be present.

Preferably, the ligation reaction is in the presence of a crowding agent. A crowding agent increases the likelihood of the adaptor and elongation product interacting with each other by decreasing the effective reactive volume, see Zimmerman et al., Proc Natl Acad Sci U S A. 1983; 80(19):5852-6. Further crowding agents are e.g. disclosed in US 5,554,730, US 8,017,339 and WO 2013/038010 A2. Preferably, the crowding agent is a macromolecule, polymer or polymer comprising compound, like a polyalkyl glycol, preferably PEG, Octoxinol or Triton X, or a polysorbate, preferably Tween. In preferred embodiments, the crowding agent is used in concentrations of 5% to 35% (v/v), especially preferred 10% to 25% (v/v). Preferably, the crowding agent has a molecular weight of 200 bis 35000 g/mol, preferably 1000 to 10000 g/mol. In particular preferred is a polyalkyl glycol, like PEG, especially with said molecular weight. A crowding agent is preferably provided in the inventive kit, preferably in a ligation buffer.

Other ingredients for the kit, in any component, are buffers, salts, enzymatic cofactors and metals, such as Mn²⁺ and Mg²⁺ for polymerases and ligases, solvents, containers.

The present invention provides a kit for performing the inventive method. Such a kit may comprise any of the compounds and means described so far. Preferably the kit comprises (i) at least one oligonucleotide primer capable of hybridizing to a template nucleic acid and priming an elongation reaction on its 3' end, (ii) one or more elongation stoppers capable of hybridizing to a template nucleic acid, preferably capable of priming an elongation reaction on its 3' end, (iii) one or more adaptor nucleic acids that comprise an identification sequence on its 5' end, wherein said identification sequence does not hybridize to the elongation stopper, preferably wherein the adaptor nucleic acid is bound to, hybridized to or is not bound to an elongation stopper, (iv) a reverse transcriptase, and (v) an oligonucleotide ligase, (iv) and (v) may be optional since they might be available to many laboratories independent of the present invention. The important parts are the adaptor/stopper designs, in particular the identification sequences on the adaptors. Preferably a plurality of adaptors with different identification sequences are provided in the kit - as described above. All these components the kit have been described above and any preferred embodiment thereof also applies to the kit as well. Preferably the kit comprises at least 10, more preferred at least 50, adaptor nucleic acids with different identification sequences. The reasons for such a preferred embodiment have been given above. Preferably the oligonucleotide primer comprises an annealing sequence for annealing to the template, which comprises an oligo(dT) sequence for annealing to an oligo(A) sequence in the template, preferably wherein said oligo(dT) sequence comprises one or more 3' anchoring nucleotides different from the oligo (dT) sequence. The kit may also comprise a solid phase for purification, such as beads, preferably magnetic beads (see method details above, which also read on the kit components' suitability and embodiments).

All preferred embodiments as described above can be combined. Such a method uses a random primer (with a linker sequence) that is also a stopper (also called "Strand Displacement Stop Primer"). After the elongation reaction, preferably a purification of the elongation products (hybridized to the template) is done to remove unbound primers and stoppers. Then adaptors with their linkers and identification sequences are ligating to the elongation product. The identification sequence has a random sequence with a length of preferably between 4 and 12 nt. One preferred option is to use mixtures of differently long identification sequences because ligases tend to impose ligation biases by favouring certain 5' located nucleotides in the ultimate and penultimate position. Because such biases can affect read quality in sequencing such mixtures equalize the nucleotide distribution when sequencing across the region of ligation junctions. However, the variable identification sequence provides a much more unbiased ligation as any other determined sequence and serves at the same time also as a UMI (Unique Molecular Index). A identification sequences, such as UMI, allows to determine if sequencing reads which possess an identical sequence, or which map to an identical position in a reference annotation which accounts for minor sequencing errors,are coming from different template molecules or from one template molecule and are just the result of further amplification (PCR duplication). The adaptor is hybridized to the primer when present.

Identification sequences, like UMIs, can also distinguish between real SNPs (single nucleotide polymorphisms) between individuals and errors (mutations) introduced during reverse transcription or in early PCR cycles, which are amplified later. All of those randomly occurring and amplified errors should have the same identifier, whereas "real SNPs" ins a sample have various different identifiers. Or RNA-editing events that introduce modified bases leading to mis-incorporation and thus errors during RT could be more reliably quantified.

Identification sequences, like UMIs, could also be used to reliably determine and quantify allele frequencies in populations, molecular markers and causative mutations in hereditary diseases. Preferably DNA templates are used for this embodiment.

A further preferred combination is a method of the invention wherein at least one, preferably at least 9, elongation stopper has primer activity and is also elongated during the elongating step and at least two, preferably at least 10, adaptor nucleic acids that comprise different identification sequences are used, whereby at least two, preferably at least 10, different labelled fragments are generated, optionally amplifying the labelled fragments, further comprising assembling the sequences of amplification fragments which are unique, wherein the labels are used to identify unique amplification fragments. The different labels in the amplified labelled fragments can be used for identifying unique amplification fragments.

A further preferred method uses stoppers with primer functions. Preferably a plurality of such primers is used. In such a method, without differentiating between stoppers and primers, an embodiment of the invention can be defined as follows: A method for generating labelled amplification fragments of a nucleic acid template comprising the steps of providing said template nucleic acid, annealing a plurality of oligonucleotide primers to said template nucleic acid, elongating the oligonucleotide primers in a template specific manner thereby creating a plurality of elongation products, wherein said elongating reactions stop when the elongation products reach the 5' end of the template nucleic acid or an oligonucleotide primer that is annealed to the template nucleic acid downstream of such an elongation product, providing a plurality of adaptor nucleic acids that comprise an identification sequence on their 5' ends, wherein said identification sequences do not hybridize to the oligonucleotide primer or to the template, ligating the adaptor nucleic acids of the plurality at their respective 5' ends to the 3' end of the elongation products, thereby generating plurality of labelled amplification fragments. This is a preferred embodiment that can be combined with any particularly described aspects in the claims and described above. Everything described above for stoppers applies to the primers in this embodiment since these primers are stoppers with primer function. The term "plurality" is used for oligonucleotide primers, elongation products (which are the result of the primers' elongation), adaptor nucleic acids and labelled amplification fragments (which are the result of elongation and adaptor ligation). As indicated, the amount of some of these pluralities is a result of the method. The amounts of oligonucleotide primers and adaptor nucleic acids can be selected - as described above. Their amounts can be selected independently but are preferably approximately the same for pairwise association with a given elongation product. Preferably the plurality is e.g. 10 or more, 50 or more, 100 or more, 200 or more, etc. Many different oligonucleotide primers and adaptor nucleic acids can be used: For the oligonucleotide primers to bind to multiple different locations on the template, for the adaptor nucleic acids to have different identification sequences, preferably unique identification sequences for the labelled amplification fragments. Although in this embodiment primers and stopper are the same, a special primer that does not need (but may have) stopper function may also be added, such as a 5' end specific primer, like an oligo(A) targeting primer as described above.

The present invention is further described in the following figures and examples, without being limited to these embodiments of the present invention.

### Figures:

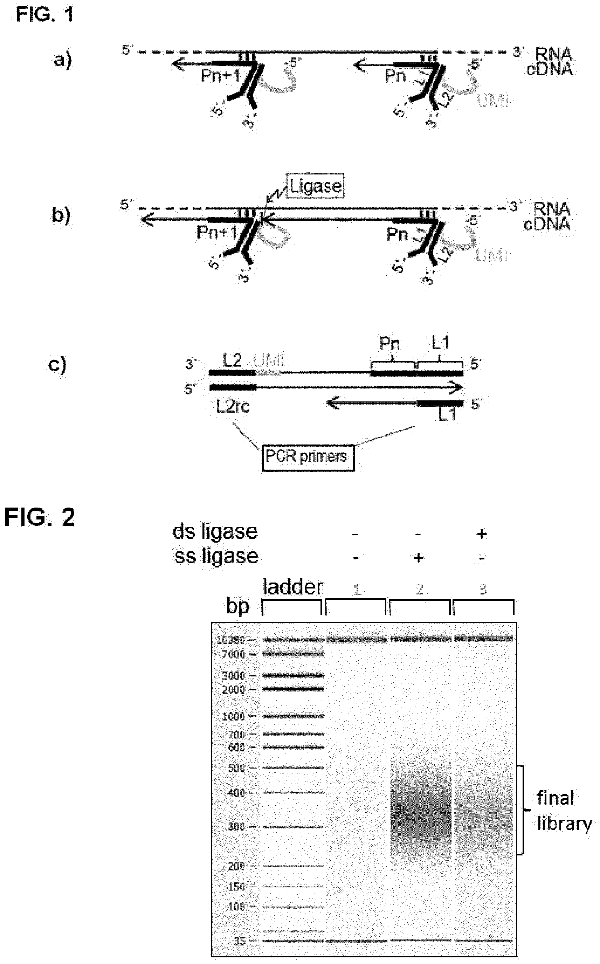

**Figure 1****:** Schematic representation of creating a UMI-linker tagged short cDNA library using a primer with SDS properties and a partially complementary UMI-containing linker oligo within the body of the RNA.
   a) General strand displacement stop primers Pn are hybridized to an RNA transcript, with primer Pn+1 hybridized to a more upstream (5') position of the template RNA than primer Pn. When the reverse transcriptase while extending Pn reaches a primer Pn+1, the polymerase reaction will be stopped by the strand displacement stop technology described in WO 2013/038010 A2. A UMI-containing linker oligo encompassing L2 which is complementary to L1 is hybridized to primers Pn and Pn+1. b) During ligation the extension product is now ligated to the UMI preceding the L2 strand of the linker. In this manner again, a cDNA library is created that has two linker sequences (L1, L2) present on its ends and contains unique molecular identifiers. c) Finally, a PCR is performed to amplify these libraries.
**Figure 2****:** Generation of UMI-containing libraries Figure 2 shows libraries generated by the SDS/ligation approach. Ligation of the UMI-containing partially complementary L2 linker (see Fig. 1 for reference) can be performed using either a ss ligase or a ds ligase (lane 2, 3). No libraries are generated when ligase is omitted (lane 1). After ligation, cDNA fragments containing L1 and L2 linkers are amplified by PCR and analysed. Shown are gel images from an HS DNA Assay run on a Bioanalyzer (Agilent Technologies, Inc.).

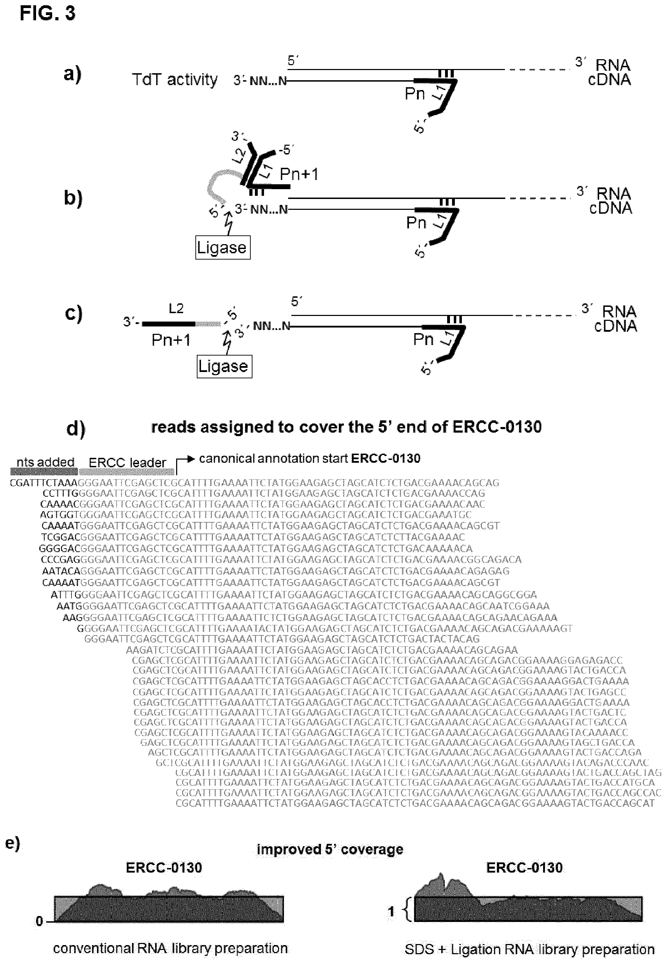

**Figure 3****:** Improved 5' end coverage of transcripts achieved by ligation of L2 linkers to cDNA at the 5' end of the RNA template.
   a) Schematic representation of the RT reaction at 5' end of transcripts. Without SDS by downstream primers Pn+1, the terminal deoxynucleotidyl transferase activity (TdT) of the RT adds untemplated nucleotides to the 3' end of the cDNA generating an overhang. b) the non-templated nts can either serve as hybridisation site for L1 containing primer Pn+1. In conjunction with partially hybridized L2, the ligation of the UMI-L2 linker can occur in a double strand. c) Alternatively, in the absence of priming the UMI-L2 linker can be ligated as a single strand. d) Libraries generated as shown schematically in Fig. 3 a-c) were sequenced on an Illumina NextSeq 500 (single read, 75bp). Shown are reads mapping to the 5' end of ERCC-0130 (as present in SIRV set 3, Lexogen Catalog #051.0N). Reads were analyzed without trimming of additional and miss-matching bases. Nucleotides marked in gray correspond to the annotation of ERCC-0130, and nucleotides shown in black are derived from non-templated addition by TdT activity of the RT. Read sequences are SEQ ID NO: 11 to 41, from top to bottom. e) Improved 5' end coverage of the SDS/ligation approach as compared to conventional protocols. Libraries were prepared using a conventional protocol (NEBNext® Ultra™ II directional RNA Library Prep Kit for Illumina®, New England Biolabs, Catalog # E7760S) or the SDS/ligation approach and sequenced on an Illumina NextSeq 500 (paired end read, 150bp). Reads mapping to ERCC-0130 were superimposed and compared with expected coverage shown as rectangles, left: conventional RNA library preparation protocol, right: coverage obtained by the novel SDS/ligation technology.

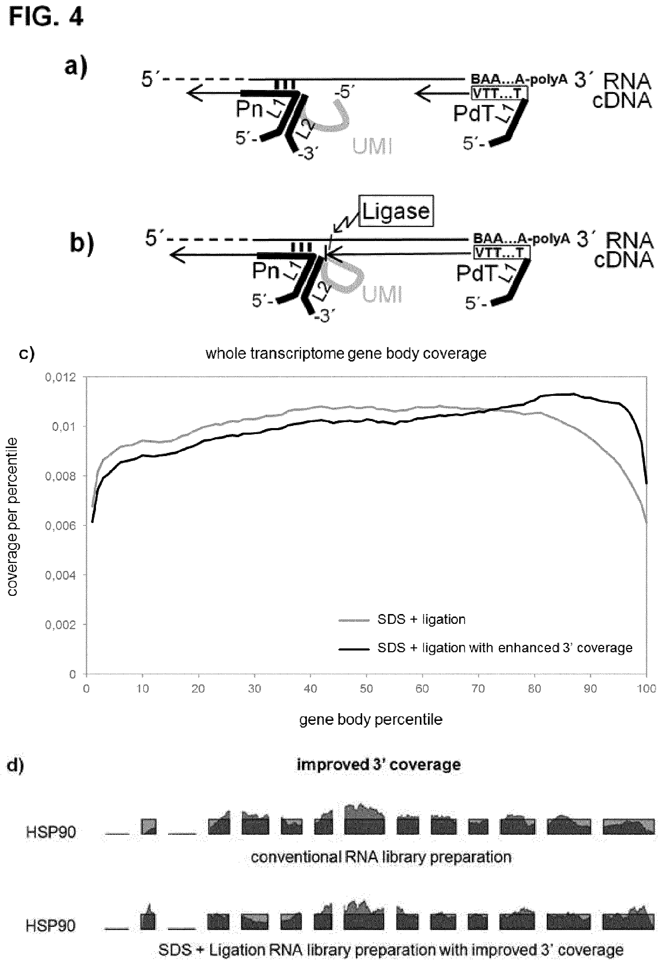

**Figure 4****:** Schematic representation of the reaction used to improve 3' end coverage by the SDS/ligation approach and a combination of general (Pn) and oligo-dT primers (PdT).
   a) General primer Pn is hybridized to the RNA template within the body of the RNA. Additionally, present oligo-dT primers (PdT) hybridize to the poly(A) tail of the 3' end of polyadenylated transcripts. RT will extend PdT until a downstream primer Pn will be reached and stops strand displacement.
   b) During ligation, the UMI-containing L2 linker will be ligated to cDNA fragments spanning the 3' end, generating L1 and L2-linked, UMI-containing cDNA libraries covering the 3' ends of transcripts. c) gene body coverage plot showing enhanced coverage of the 3' end of transcripts over the whole transcriptome. Libraries were prepared using the SDS + ligation protocol using a mixture of random priming and oligo-dT first strand synthesis primer as described in example 3. Libraries were sequenced on a NextSeq 500 machine and gene body coverage over the transcriptome was plotted in comparison to the previously described SDS + ligation protocol. d) exemplary coverage over an endogenous housekeeping gene (HSP90) for a conventional library preparation method (upper panel) and the SDS + ligation protocol with oligo-dT titration (lower panel) which results in improved 3' end coverage.

### Examples:

### Example 1: Ligation of unique molecular identifiers (UMI) to first strand cDNA fragments.

Libraries were prepared from universal human reference RNA (Agilent Technologies, Catalog # 740000) containing SIRV Set 3 spike in control mix (Lexogen, Catalog # 051.0N) according to the manufacturer's instruction.

After cDNA synthesis, downstream primers (Pn+1 (L2)) containing a unique molecular identifier of a length between 2 and 24 nucleotides, preferably between 6 and 12 nucleotides, can be ligated to the newly transcribed cDNA strand in the hybrid with the template RNA. Reverse transcription was performed using oligos, template and conditions as described WO 2013/038010 A2. Various Ligases and combinations thereof can be used to ligate oligos like:
SEQ ID No: 1: (Phos)(5'-NNNNNNAGATCGGAAGAGCACACGTCTGAACTCCAGTCAC -3' (3InvdT)),
SEQ ID No: 2: (Phos)(5'-NNNNNNNNNNAGATCGGAAGAGCACACGTCTGAA-3' (3InvdT)),
SEQ ID No: 3: (Phos) (5'-NNNNNNNNNNAGATCGGAAGAGCGTCGTGTAGGGAAAGAGTG-3' (3InvdT)),
SEQ ID No: 4: (Phos)(5'-NNNNNNNNNNAGATCGGAAGAGCGTCGTGTAGG-3' (3InvdT)),
SEQ ID No: 5: (Phos) (5'-NNNNNNNNNNNAGATCGGAAGAGCGTCGTGTAGGGAAAGAGTG-3' (3InvdT)),
SEQ ID No: 6: (Phos) (5'-NNNNNNNNNNNNAGATCGGAAGAGCGTCGTGTAGGGAAAGAGTG-3' (3InvdT)),
SEQ ID No: 7: (Phos)(5'-NNNNNNNNNNNNAGATCGGAAGAGCGTCGTGTAGG-3' (3InvdT)),
SEQ ID No: 8: (Phos)(5'-+NNNNNNNNNNAGATCGGAAGAGCGTCGTGTAGG-3' (3InvdT)),
SEQ ID No: 9: (Phos)(5'-+NNNNNNNNNNNNAGATCGGAAGAGCGTCGTGTAGG-3' (3InvdT)).

After reverse transcription (RT) the samples were purified by solid phase reverse immobilization (SPRI) with magnetic purification beads (AMPure Beads; Agentcourt) according to the manufacturer's instruction. The cDNA:RNA hybrids were eluted in 20 µl water or 10 mM Tris, pH 8.0, before 17 µl of the supernatants were transferred into a new PCR plate. Then, ligation reactions were performed in 60 µl with 20% PEG-8000, 50 mM Tris-HCl (pH 7.5 at 25°C), 10 mM MgCl₂, 5 mM DTT, 0.4 mM ATP, 0.01% Triton-x100, 50 pg/ml BSA and 20 units ligase, which can either be a single-strand specific ligase and/or a double strand-specific ligase. Un-ligated small fragments and remaining oligos were removed by SPRI purification. All remaining primary cDNA libraries were amplified in a PCR reaction using a high-fidelity polymerase, and the following program: 98°C for 30 seconds followed by 10-25 PCR cycles of 98°C for 10 seconds, 65°C for 20 seconds and 72°C for 30 seconds. Final extension was performed at 72°C for 60 seconds. Figure 18 shows the general principle underlying the ligation of the extended cDNA to the UMI-containing linker oligo (L2) which has a complementary sequence to the strand displacement stop primer (L1).

The example in Figure 2 shows that various ligases can perform the ligation reaction of a UMI containing oligo nucleotide, and thus produce cDNA fragments that contain both PCR linkers and are amplifiable by PCR (Fig. 2, lane 2-3). In contrast, the control experiment omitting any ligase shows that no libraries can be amplified emphasizing the specificity of the reaction (Fig. 2, lane 1).

### Example 2: Improved 5' end coverage as a consequence of terminal transferase activity and ss-ligation of a UMI-linker to first strand cDNA fragments.

Libraries were prepared from universal human reference RNA (Agilent Technologies, Catalog # 740000) containing SIRV Set 3 spike in control mix (Lexogen, Catalog # 051.0N) according to the manufacturer's instruction.

First strand cDNA synthesis stops at the 5' ends of template RNA molecules. Terminal transferase activity of reverse transcriptases catalyzes non-templated addition of nucleotides at the 3' end of the cDNA stand (Fig. 3 a).

Ligation of UMI-linker oligos (e.g., SEQ IDs 1-9) after reverse transcription can occur in double strand formation (Fig.3 b) and at single-stranded overhangs (Fig.3 c). Following SPRI-purification and PCR amplification, libraries were sequenced on a NextSeq 500, either in single read or paired-end mode. Reads mapping to the 5' end of ERCC-0130 were analysed without prior clipping of miss-matched nucleotides. Reads covering the 5' end of ERCC-0130 are shown exemplarily in Fig. 3 d. Addition of terminal nucleotides and the UMI ligation at extended single strands result in improved 5' coverages. The comparison of coverage profiles between common RNA-seq library preparation and the present invention are shown in Fig. 3 e. Coverages are seen as superposition of all aligned reads (trace shown in grey) and compared to the expected uniform coverage shown as rectangle. Whereas in sequencing data derived from conventional protocols the 5' and 3' ends are less efficiently covered apparent in a slope towards either end (Fig. 3 e, left), the novel protocol generates more reads mapping to the extreme 5' end of transcripts (Fig. 3 e, right).

### Example 3: Improvement of 3' end coverage by titration of oligo-dT first strand synthesis primers.

The coverage of transcript 3' ends can be modified, preferably increased, by using oligo-dT containing first strand primers (Pn containing L1) which are added to the mixture of random-priming SDS oligos, which contain already a portion of T-rich, and T-only priming sequences (such as SEQ ID No: 10 5'-GTGACTG-GAGTTCAGACGTGTGCTCTTCCGATCT +TTT TTT TTT TTT TTT TTT+ V-3') according to the normal distribution of random nucleotides, to boost the coverage at the 3' ends. Depending on the chosen ratio between random and poly-dT L1 primers the change in sequencing depth at the 3'-end sites can be foregrounded (Fig. 4). The ratios of random SDS primers and specific oligo dT primers, as well as the primer length and LNA content can vary, and will determine the amount of over-representation of the 3' ends.

Libraries were prepared by SDS + ligation using either random priming displacement stop primers only or a mixture with various amounts of oligo-dT first strand primers (SEQ ID No:10). The resulting libraries were subjected to sequencing on a NextSeq 500, data was analysed, and gene body coverage plots over the whole transcriptome were generated from mapped reads using the geneBody_coverage python script available from rseqc (Fig. 4 c). The coverage of 3' ends can be significantly increased upon addition of oligo-dT primers during reverse transcription.

Further, gene coverages were visualized exemplarily for endogenous genes using a custom script to evaluate coverage on individual genes. Figure 4 d shows the coverage of housekeeping gene HSP90 obtained by a conventional RNA library preparation protocol (upper panel) with notoriously under-represented 5' and 3' ends. In contrast, the SDS-ligation protocol with oligo-dT titration shows an improved 5' and 3' coverage (lower panel).

## Claims

1. A method for generating labelled amplification fragments of a nucleic acid template comprising the steps of
providing said template nucleic acid,
annealing at least one oligonucleotide primer to said template nucleic acid,
elongating the at least one oligonucleotide primer in a template specific manner thereby creating an elongation product, wherein said elongating reaction stops when the elongation product reaches the 5' end of the template nucleic acid or a nucleic acid elongation stopper that is annealed to the template nucleic acid downstream of the elongation product,
providing an adaptor nucleic acid that comprises an identification sequence on its 5' end, wherein said identification sequence does not hybridize to the elongation stopper or to the template,
ligating the adaptor nucleic acid at its 5' end to the 3' end of the elongation product, thereby generating a labelled amplification fragment.

2. The method of claim 1, wherein in case the elongation product reaches the 5' end of the template nucleic acid, a nucleotide polymerase is allowed to add untemplated nucleotides to the elongation product, preferably by a terminal transferase activity of the polymerase, and/or preferably wherein 1 to 15 untemplated nucleotides are added in at least 70% of the extension products.

3. The method of claim 1 or 2, wherein a plurality of adaptor nucleic acids is provided and used in the ligation step, wherein said adaptors of the plurality have different identification sequences, preferably wherein at least 10, more preferred at least 50, adaptor nucleic acids with different identification sequences are provided and used in the ligation step.

4. The method of any one of claims 1 to 3, wherein the identification sequence is a random sequence.

5. The method of any one of claims 1 to 4, wherein the elongation stopper has primer activity and is also elongated during the elongating step, preferably wherein at least 9, more preferred at least 49, elongation stoppers are used that have different annealing sequences for annealing to the template and thereby potentially anneal to different locations on the template nucleic acid.

6. The method of claim 5, wherein the annealing sequence is a random sequence.

7. The method of any one of claims 1 to 6, wherein the adaptor nucleic acid(s) is/are bound to, hybridized to or is/are not bound to the elongation stopper(s), preferably when the adaptor nucleic acids are bound to or hybridized to the elongation stoppers then the identification sequence is independent of an annealing sequence of the elongation stopper for annealing the elongation stopper to the template.

8. The method of any one of claims 1 to 7, wherein the template is RNA, preferably wherein a reverse transcriptase is used for elongation.

9. The method of any one of claims 1 to 8, wherein the oligonucleotide primer and preferably also the elongation stopper comprises a universal amplification sequence and/or wherein the adaptor nucleic acid comprises a universal adaptor amplification sequence.

10. The method of any one of claims 1 to 9, wherein the oligonucleotide primer comprises an annealing sequence for annealing to the template, which comprises an oligo (T) sequence for annealing to an oligo (A) sequence in the template, preferably wherein said oligo (T) sequence comprises one or more 3' anchoring nucleotides different from the oligo (T) sequence.

11. The method of any one of claims 1 to 10, wherein the ligation reaction is in the presence of a crowding agent, preferably a polymer or polymer comprising compound, like a polyalkyl glycol, preferably PEG, Octoxinol or Triton X, or a polysorbate, preferably Tween; and/or wherein the elongation stopper and preferably also the oligonucleotide primer comprise(s) one or more modified nucleotide(s) that increase the melting temperature in an annealing sequence for annealing to the template.

12. The method of any one of claims 1 to 11, wherein at least one, preferably at least 9, elongation stopper has primer activity and is also elongated during the elongating step and at least two, preferably at least 10, adaptor nucleic acids that comprise different identification sequences are used, whereby at least two, preferably at least 10, different labelled amplification fragments are generated, optionally amplifying the labelled amplification fragments, further comprising assembling the sequences of amplification fragments which are unique, wherein the labels are used to identify unique amplification fragments.

13. A kit for performing a method of any one of claims 1 to 12, comprising
at least one oligonucleotide primer capable of hybridizing to a template nucleic acid and priming an elongation reaction on its 3' end,
one or more elongation stoppers capable of hybridizing to a template nucleic acid, preferably capable of priming an elongation reaction on its 3' end,
one or more adaptor nucleic acids that comprise an identification sequence on its 5' end, wherein said identification sequence does not hybridize to the elongation stopper, preferably wherein the adaptor nucleic acid is bound to, hybridized to or is not bound to an elongation stopper,
a reverse transcriptase, and an oligonucleotide ligase.

14. The kit of claim 13 comprising at least 10, more preferred at least 50, adaptor nucleic acids with different identification sequences.

15. The kit of claim 13 or 14, wherein at least one oligonucleotide primer comprises an annealing sequence for annealing to the template, which comprises an oligo (T) sequence for annealing to an oligo (A) sequence in the template, preferably wherein said oligo (T) sequence comprises one or more 3' anchoring nucleotides different from the oligo (T) sequence.


SEQUENCE LISTING

<110>  LEXOGEN GMBH

<120> Nucleic acid amplification and identification method

<130> R 73847

<160> 41

<170> BiSSAP 1.3

<210> 1
<211> 41
<212> DNA
<213> Artificial Sequence

<220>
<223> oligo

<220>
<221> modified_base
<222> 1
<223> /mod_base="OTHER"
      /note="5' phosphorylated; a or g or c or t"

<220>
<221> misc_difference
<222> 2,3,4,5,6
<223> /note="a or g or c or t"

<220>
<221> modified_base
<222> 41
<223> /mod_base="OTHER"
      /note="3' Inverted dT (reverse linkage)"

<400> 1
nnnnnnagat cggaagagca cacgtctgaa ctccagtcac n                    41


<210> 2
<211> 35
<212> DNA
<213> Artificial Sequence

<220>
<223> oligo

<220>
<221> modified_base
<222> 1
<223> /mod_base="OTHER"
      /note="5' phosphorylated; a or g or c or t"

<220>
<221> misc_difference
<222> 2,3,4,5,6,7,8,9,10
<223> /note="a or g or c or t"

<220>
<221> modified_base
<222> 35

<223> /mod_base="OTHER"
      /note="3' Inverted dT (reverse linkage)"

<400> 2
nnnnnnnnnn agatcggaag agcacacgtc tgaan                                35


<210> 3
<211> 43
<212> DNA
<213> Artificial Sequence

<220>
<223> oligo

<220>
<221> modified_base
<222> 1
<223> /mod_base="OTHER"
      /note="5' phosphorylated; a or g or c or t"

<220>
<221> misc_difference
<222> 2,3,4,5,6,7,8,9,10
<223> /note="a or g or c or t"

<220>
<221> modified_base
<222> 43
<223> /mod_base="OTHER"
      /note="3' Inverted dT (reverse linkage)"

<400> 3
nnnnnnnnnn agatcggaag agcgtcgtgt agggaaagag tgn                       43


<210> 4
<211> 34
<212> DNA
<213> Artificial Sequence

<220>
<223> oligo

<220>
<221> modified_base
<222> 1
<223> /mod_base="OTHER"
      /note="5' phosphorylated; a or g or c or t"

<220>
<221> misc_difference
<222> 2,3,4,5,6,7,8,9,10
<223> /note="a or g or c or t"

<220>
<221> modified_base
<222> 34
<223> /mod_base="OTHER"
      /note="3' Inverted dT (reverse linkage)"

<400> 4
nnnnnnnnnn agatcggaag agcgtcgtgt aggn                                 34

<210> 5
<211> 44
<212> DNA
<213> Artificial Sequence

<220>
<223> oligo

<220>
<221> modified_base
<222> 1
<223> /mod_base="OTHER"
      /note="5′ phosphorylated; a or g or c or t"

<220>
<221> misc_difference
<222> 2,3,4,5,6,7,8,9,10,11
<223> /note="a or g or c or t"

<220>
<221> modified_base
<222> 44
<223> /mod_base="OTHER"
      /note="3′ Inverted dT (reverse linkage)"

<400> 5
nnnnnnnnnn nagatcggaa gagcgtcgtg tagggaaaga gtgn                        44


<210> 6
<211> 45
<212> DNA
<213> Artificial Sequence

<220>
<223> oligo

<220>
<221> modified_base
<222> 1
<223> /mod_base="OTHER"
      /note="5′ phosphorylated; a or g or c or t"

<220>
<221> misc_difference
<222> 2,3,4,5,6,7,8,9,10,11,12
<223> /note="a or g or c or t"

<220>
<221> modified_base
<222> 45
<223> /mod_base="OTHER"
      /note="3′ Inverted dT (reverse linkage)"

<400> 6
nnnnnnnnnn nnagatcgga agagcgtcgt gtagggaaag agtgn                       45


<210> 7
<211> 36
<212> DNA

<213> Artificial Sequence

<220>
<223> oligo

<220>
<221> modified_base
<222> 1
<223> /mod_base="OTHER"
      /note="5′ phosphorylated; a or g or c or t"

<220>
<221> misc_difference
<222> 2,3,4,5,6,7,8,9,10,11,12
<223> /note="a or g or c or t"

<220>
<221> modified_base
<222> 36
<223> /mod_base="OTHER"
      /note="3′ Inverted dT (reverse linkage)"

<400> 7
nnnnnnnnnn nnagatcgga agagcgtcgt gtaggn                                36


<210> 8
<211> 34
<212> DNA
<213> Artificial Sequence

<220>
<223> oligo

<220>
<221> modified_base
<222> 1
<223> /mod_base="OTHER"
      /note="5′ phosphorylated; a or g or c or t"

<220>
<221> misc_difference
<222> 2,3,4,5,6,7,8,9,10
<223> /note="a or g or c or t"

<220>
<221> modified_base
<222> 34
<223> /mod_base="OTHER"
      /note="3′ Inverted dT (reverse linkage)"

<400> 8
nnnnnnnnnn agatcggaag agcgtcgtgt aggn                                  34


<210> 9
<211> 36
<212> DNA
<213> Artificial Sequence

<220>
<223> oligo

<220>
<221> modified_base
<222> 1
<223> /mod_base="OTHER"
      /note="5′ phosphorylated; a or g or c or t"

<220>
<221> misc_difference
<222> 2,3,4,5,6,7,8,9,10,11,12
<223> /note="a or g or c or t"

<220>
<221> modified_base
<222> 36
<223> /mod_base="OTHER"
      /note="3′ Inverted dT (reverse linkage)"

<400> 9
nnnnnnnnnn nnagatcgga agagcgtcgt gtaggn                                36


<210> 10
<211> 53
<212> DNA
<213> Artificial Sequence

<220>
<223> oligo

<400> 10
gtgactggag ttcagacgtg tgctcttccg atcttttttt tttttttttt ttv             53


<210> 11
<211> 75
<212> DNA
<213> Artificial Sequence

<220>
<223> sequencing read

<400> 11
cgatttctaa agggaattcg agctcgcatt ttgaaaattc tatggaagag ctagcatctc      60

tgacgaaaac agcag                                                       75


<210> 12
<211> 68
<212> DNA
<213> Artificial Sequence

<220>
<223> sequencing read

<400> 12
cctttgggga attcgagctc gcattttgaa aattctatgg aagagctagc atctctgacg      60

aaaaccag                                                               68


<210> 13
<211> 68

<212> DNA
<213> Artificial Sequence

<220>
<223> sequencing read

<400> 13
caaaacggga attcgagctc gcattttgaa aattctatgg aagagctagc atctctgacg     60

aaaacaac                                                              68


<210> 14
<211> 66
<212> DNA
<213> Artificial Sequence

<220>
<223> sequencing read

<400> 14
agtggtggga attcgagctc gcattttgaa aattctatgg aagagctagc atctctgacg     60

aaatgc                                                                66


<210> 15
<211> 70
<212> DNA
<213> Artificial Sequence

<220>
<223> sequencing read

<400> 15
caaaatggga attcgagctc gcattttgaa aattctatgg aagagctagc atctctgacg     60

aaaacagcgt                                                            70


<210> 16
<211> 65
<212> DNA
<213> Artificial Sequence

<220>
<223> sequencing read

<400> 16
tcggacggga attcgagctc gcattttgaa aattctatgg aagagctagc atctcttacg     60

aaaac                                                                 65


<210> 17
<211> 66
<212> DNA
<213> Artificial Sequence

<220>
<223> sequencing read

<400> 17

ggggacggga attcgagctc gcattttgaa aattctatgg aagagctagc atctctgaca 60

aaaaca 66

<210> 18
<211> 73
<212> DNA
<213> Artificial Sequence

<220>
<223> sequencing read

<400> 18
cccgagggga attcgagctc gcattttgaa aattctatgg aagagctagc atctctgacg 60

aaaacggcag aca 73

<210> 19
<211> 71
<212> DNA
<213> Artificial Sequence

<220>
<223> sequencing read

<400> 19
aatacaggga attcgagctc gcattttgaa aattctatgg aagagctagc atctctgacg 60

aaaacagaga g 71

<210> 20
<211> 70
<212> DNA
<213> Artificial Sequence

<220>
<223> sequencing read

<400> 20
caaaatggga attcgagctc gcattttgaa aattctatgg aagagctagc atctctgacg 60

aaaacagcgt 70

<210> 21
<211> 74
<212> DNA
<213> Artificial Sequence

<220>
<223> sequencing read

<400> 21
atttggggaa ttcgagctcg cattttgaaa attctatgga agagctagca tctctgacga 60

aaacagcagg cgga 74

<210> 22
<211> 75

<212> DNA
<213> Artificial Sequence

<220>
<223> sequencing read

<400> 22
aatggggaat tcgagctcgc attttgaaaa ttctatggaa gagctagcat ctctgacgaa      60

aacagcaatc ggaaa                                                        75


<210> 23
<211> 75
<212> DNA
<213> Artificial Sequence

<220>
<223> sequencing read

<400> 23
aaggggaatt cgagctcgca ttttgaaaat tctctggaag agctagcatc tctgacgaaa      60

acagcagaac agaaa                                                        75


<210> 24
<211> 75
<212> DNA
<213> Artificial Sequence

<220>
<223> sequencing read

<400> 24
ggggaattcg agctcgcatt ttgaaaatac tatggaagag ctagcatctc tgacgaaaac      60

agcagacgaa aaagt                                                        75


<210> 25
<211> 61
<212> DNA
<213> Artificial Sequence

<220>
<223> sequencing read

<400> 25
gggaattcga gctcgcattt tgaaaattct atggaagagc tagcatctct gactactaca      60

g                                                                       61


<210> 26
<211> 60
<212> DNA
<213> Artificial Sequence

<220>
<223> sequencing read

<400> 26

aagatctcgc attttgaaaa ttctatggaa gagctagcat ctctgacgaa aacagcagaa 60

<210> 27
<211> 74
<212> DNA
<213> Artificial Sequence

<220>
<223> sequencing read

<400> 27
cgagctcgca ttttgaaaat tctatggaag agctagcatc tctgacgaaa acagcagacg 60

gaaaaggaga gacc 74

<210> 28
<211> 75
<212> DNA
<213> Artificial Sequence

<220>
<223> sequencing read

<400> 28
cgagctcgca ttttgaaaat tctatggaag agctagcatc tctgacgaaa acagcagacg 60

gaaaagtact gacca 75

<210> 29
<211> 75
<212> DNA
<213> Artificial Sequence

<220>
<223> sequencing read

<400> 29
cgagctcgca ttttgaaaat tctatggaag agctagcacc tctgacgaaa acagcagacg 60

gaaaaggact gaaaa 75

<210> 30
<211> 75
<212> DNA
<213> Artificial Sequence

<220>
<223> sequencing read

<400> 30
cgagctcgca ttttgaaaat tctatggaag agctagcatc tctgacgaaa acagcagacg 60

gaaaagtact gagcc 75

<210> 31
<211> 75
<212> DNA
<213> Artificial Sequence

<220>
<223> sequencing read

<400> 31
cgagctcgca ttttgaaaat tctatggaag agctagcacc tctgacgaaa acagcagacg     60

gaaaaggact gaaaa                                                      75


<210> 32
<211> 75
<212> DNA
<213> Artificial Sequence

<220>
<223> sequencing read

<400> 32
cgagctcgca ttttgaaaat tctatggaag agctagcatc tctgacgaaa acagcagacg     60

gaaaagtact gactc                                                      75


<210> 33
<211> 75
<212> DNA
<213> Artificial Sequence

<220>
<223> sequencing read

<400> 33
cgagctcgca ttttgaaaat tctatggaag agctagcatc tctgacgaaa acagcagacg     60

gaaaagtact gacca                                                      75


<210> 34
<211> 75
<212> DNA
<213> Artificial Sequence

<220>
<223> sequencing read

<400> 34
cgagctcgca ttttgaaaat tctatggaag agctagcatc tctgacgaaa acagcagacg     60

gaaaagtaca aaacc                                                      75


<210> 35
<211> 75
<212> DNA
<213> Artificial Sequence

<220>
<223> sequencing read

<400> 35
gagctcgcat tttgaaaatt ctatggaaga gctagcatct ctgacgaaaa cagcagacgg     60

aaaagtagct gacca                                                    75


<210> 36
<211> 75
<212> DNA
<213> Artificial Sequence

<220>
<223> sequencing read

<400> 36
agctcgcatt ttgaaaattc tatggaagag ctagcatctc tgacgaaaac agcagacgga      60

aaagtactga ccaga                                                    75


<210> 37
<211> 75
<212> DNA
<213> Artificial Sequence

<220>
<223> sequencing read

<400> 37
gctcgcattt tgaaaattct atggaagagc tagcatctct gacgaaaaca gcagacggaa      60

aagtacagac ccaac                                                    75


<210> 38
<211> 74
<212> DNA
<213> Artificial Sequence

<220>
<223> sequencing read

<400> 38
cgcattttga aaattctatg gaagagctag catctctgac gaaaacagca gacggaaaag      60

tactgaccag ctag                                                     74


<210> 39
<211> 73
<212> DNA
<213> Artificial Sequence

<220>
<223> sequencing read

<400> 39
cgcattttga aaattctatg gaagagctag catctctgac gaaaacagca gacggaaaag      60

tactgaccat gca                                                      73


<210> 40
<211> 74
<212> DNA
<213> Artificial Sequence

<220>
<223> sequencing read

<400> 40
cgcattttga aaattctatg gaagagctag catctctgac gaaaacagca gacggaaaag     60

tactgaccag ccac                                                       74



<210> 41
<211> 73
<212> DNA
<213> Artificial Sequence

<220>
<223> sequencing read

<400> 41
cgcattttga aaattctatg gaagagctag catctctgac gaaaacagca gacggaaaag     60

tactgaccag cat                                                        73